Europäisches Patentamt

(19) European Patent Office

Office européen des brevets

(11) Publication number:

**0 286 601**
A2

(12) # EUROPEAN PATENT APPLICATION

(21) Application number: **88830116.5**

(22) Date of filing: **23.03.88**

(51) Int. Cl.⁴: **A 61 L 9/01**

(30) Priority: **07.04.87 IT 4781687**

(43) Date of publication of application:
**12.10.88 Bulletin 88/41**

(84) Designated Contracting States: **DE FR GB NL**

(71) Applicant: **Campodonico, Bruno**
**Via Fonteiana 68**
**I-00152 Rome (IT)**

(72) Inventor: **Campodonico, Bruno**
**Via Fonteiana 68**
**I-00152 Rome (IT)**

(74) Representative: **de Simone, Domenico et al**
**Ing. Barzanò & Zanardo Roma S.p.A. Via Piemonte 26**
**I-00187 Roma (IT)**

(54) **Powdered deodorant composition for shoes, In particular for sporting shoes.**

(57) Powdered deodorant composition for shoes, in particular for sporting shoes, including, mixed with other additional components, sodium bicarbonate as the active agent for deodorant effect, preferably in an amount of from 20% to 70% by weight.

EP 0 286 601 A2

Bundesdruckerei Berlin

# Description

## POWDERED DEODORANT COMPOSITION FOR SHOES, IN PARTICULAR FOR SPORTING SHOES

This invention concerns a powdered deodorant composition for shoes, in particular for sporting shoes. Specifically, the invention concerns a deodorant composition in powder to be used in shoes and specially in sporting shoes, in which the deodorant action is performed by the presence, among the other components, of sodium bicarbonate.

At present there do not exist any powdered compositions to be used for long term deodorization of shoes, in particular of sporting shoes, such as gym, tennis shoes, etc..

The problem of deodorization in said sector is at present entrusted, exclusively, to the use of deodorant insoles which contain, as their active substances, activated charcoal and similars, embedded in a base or matrix of gummy material. Said insoles however, besides being an obstacle sometimes troublesome for the foot, lose after some time their original consistence, getting soaked owing to perspiration, and therefore they do not keep their original property of odours' absorption.

Such an inconvenience evidently ends by calling for their rather frequent replacement, and it therefore entails on the user a not always negligible expense, to be cyclically repeated.

It is therefore quite evident the requirement for alternative means to be used for shoes' deodorization, mostly for the sporting ones, which may allow to avoid the mentioned inconvenience, by warranting in an advantageous way a long term effect, through a simple and economic application.

The invention intends to meet such a requirement by suggesting the use of a powdered product, in whose composition there is included an active component for shoe's deodorization.

Such a component has been found, according to the invention, to be sodium bicarbonate, to be used according to the proportions hereafter specified; on this account one should however remark that the deodorant action thus warranted is extended to the user's feet and therefore also to the socks used along with said types of shoes, unlike the means up to now used (insoles and similars), which only warrant, and unsatisfactorily too, as already pointed out, the deodorization of the shoes.

For that purpose the invention suggests the implementation of a powdered deodorant solution for shoes, in particular for sporting shoes, including, in a mixture with other components, sodium bicarbonate as the deodorant agent.

Preferably the sodium bicarbonate is contained in a proportion from 20 to 70% by weight.

In a particularly advantageous way the components being mixed with the sodium bicarbonate, are made up by one or more of the following: boric acid, magnesium carbonate, ventilated white clay, zinc oxide.

Preferably the composition according to the invention might include among said additional components some boric acid and magnesium carbonate in the percentages of 2-5% and of 10-20% respectively.

Other preferred compositions are those making use, as additional components, of the magnesium carbonate and of the ventilated white clay, in the equal percentage of 20-30% respectively.

Other compositions of special importance are also those in which, as additive components and in the per cent quantities shown, are used the following:

boric acid 2-5%
magnesium carbonate 10-20%
ventilated white clay 20-30%
zinc oxide 20-30%

As an example and not a limitation, some examples are listed hereafter of a powdered deodorant composition according to the invention:

1) Composition made up by magnesium carbonate (25%), sodium bicarbonate (50%) and ventilated white clay (25%).

2) Composition made up by sodium bicarbonate (54.5%) zinc oxide (12.72%), magnesium carbonate (15.45%), boric acid (4.54%), ventilated white clay (12.72%).

3) Composition, to be packaged in small envelopes for unit confection, including the components listed in example 2, in the same ratios, for instance: 1.50 grams of sodium bicarbonate, 0.35 grams of zinc oxide, 0.425 grams of magnesium carbonate, 0.125 grams of boric acid and 0.35 grams of ventilated white clay.

This invention has been described with special reference to its specific embodiments, but it is to be understood that modifications and changes might be adopted without consequently trespassing the relevant limits of protection.

## Claims

1. Powdered deodorant composition for shoes, in particular for sporting shoes, characterized in that it includes, mixed with other additional components, sodium bicarbonate.

2. Powdered deodorant composition according to claim 1, including sodium bicarbonate in an amount of from 20 to 70% by weight.

3. Powdered deodorant composition according to claims 1 or 2, in which said other additional components are one or more of the following:
boric acid
magnesium carbonate
ventilated white clay
zinc oxide.

4. Powdered deodorant composition according to claim 1 or 2, in which said other components are boric acid and magnesium carbonate, respectively in amounts of 2-5% and of 10-20% by weight.

5. Powdered deodorant composition according to claim 1 or 2, in which said other components are magnesium carbonate and ventilated white clay, both in the amount of 20-30% by weight.

6. Powdered deodorant composition according to claim 3, in which said additional components are present in the following amounts, by weight:

boric acid 2-5%
magnesium carbonate 10-20%
ventilated white clay 20-30%
zinc oxide 10-20%